(19) Europäisches Patentamt European Patent Office Office européen des brevets

(11) **EP 4 216 855 B1**

(12) **EUROPEAN PATENT SPECIFICATION**

(45) Date of publication and mention
of the grant of the patent:
**01.04.2026 Bulletin 2026/14**

(21) Application number: **21758706.2**

(22) Date of filing: **12.08.2021**

(51) International Patent Classification (IPC):
*A61B 18/14* (2006.01)   *A61B 18/00* (2006.01)
*A61B 90/00* (2016.01)

(52) Cooperative Patent Classification (CPC):
**A61B 18/1492;** A61B 2017/00323;
A61B 2018/00357; A61B 2018/00577;
A61B 2018/1467; A61B 2090/065

(86) International application number:
**PCT/EP2021/072485**

(87) International publication number:
**WO 2022/063482 (31.03.2022 Gazette 2022/13)**

(54) **CATHETER DEVICE WITH A SENSOR DEVICE FOR MEASURING A FORCE EFFECT**

KATHETERVORRICHTUNG MIT EINER SENSORVORRICHTUNG ZUR MESSUNG EINER KRAFTEINWIRKUNG

DISPOSITIF DE CATHÉTER DOTÉ D'UN DISPOSITIF DE CAPTEUR POUR MESURER UN EFFET DE FORCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.09.2020 EP 20197779**

(43) Date of publication of application:
**02.08.2023 Bulletin 2023/31**

(73) Proprietor: **VascoMed GmbH
79589 Binzen (DE)**

(72) Inventors:
• **KAUFMANN, Ralf**
79540 Lörrach (DE)
• **REHBERGER, Frank**
79279 Vörstette (DE)
• **KRESSMANN, Flavien**
68128 Village-N (FR)

(74) Representative: **Biotronik Corporate Services SE
Corporate Intellectual Property
Sieversufer 9
12359 Berlin (DE)**

(56) References cited:
**EP-A1- 2 491 883     US-A1- 2015 359 484**

**Description**

The invention relates to a catheter device according to the preamble of claim 1.

Such a catheter device comprises a measuring device for measuring a force effect on the catheter device. The measuring device comprises an elastically deformable transducer body with a plurality of transducer portions and a sensor device arranged on the transducer body. The transducer portions form at least one pair of adjacent transducer portions which are connected to each other via a connecting portion and are movable relative to each other with deformation of the connecting portion. The sensor device is designed to measure a force effect between the transducer portions of the at least one pair of adjacent transducer portions.

Such a catheter device may be designed as an ablation catheter, for example. An ablation catheter may be used to treat cardiac arrhythmias by selectively eliminating heart tissue, for example by heating or electrophoresis, in order to locally suppress the generation or conduction of electrical impulses. An electrode may be brought into contact with heart tissue for this purpose, for example in order to heat tissue locally by introducing a high-frequency current during high-frequency ablation and thus destroy it, or in order to induce electrophoresis in the tissue cells by means of pulsed electrical fields and thus destroy them.

In the case of such a catheter device, a force shall be reliably ascertained in order to determine whether the catheter device is in contact with the tissue to be treated and the force with which the catheter device is acting on the tissue.

In a catheter device known from US 2014/0081264 A1, an optical fibre is arranged on a transducer body, which has a plurality of sensor devices each with an optical grating. The sensor devices are intended to measure a force effect between transducer portions of the transducer body, the transducer body being designed in such a way that each sensor device may measure a force effect along a defined spatial direction.

In a catheter device known from US 2006/0200049 A1, different optical fibres are arranged on a body and each have a sensor device. A force effect may be measured via the sensor devices, which may, for example, have optical gratings in the form of Fiber Bragg Gratings. WO 2013/177577 A2 describes a catheter device which has an optical fibre enclosed in a cylindrical sleeve. A temperature-compensated Fiber Bragg Grating interferometer is optically connected to the optical fibre. When a pressure load is applied, a force value may be determined and evaluated by means of a signal generated by the interferometer.

US2015359484A1 relates to a sensor connection element of a catheter or an electrode line and to a catheter and an electrode line containing such a sensor connection element.

The object of the present invention is to provide a catheter device which enables a reliable and accurate measurement of a force effect on the catheter device. Any methods disclosed hereinafter do not form part of the scope of the invention, and are mentioned for illustrative purposes only.

This object is achieved by a subject with the features of claim 1.

Accordingly, the transducer body is made of a glass material.

Within the frame of this application glass material is to be understood as a metastable basically amorphous structure with low crystallinity comprising silicon dioxide. The amount of crystallinity is below 20%, in particular below 10%, in particular below 5%, in particular below 2%, in particular below 1%.

For example, the transducer body may be made of a quartz glass material, especially a high-purity quartz glass material.

The transducer body may also be made of a glass ceramic material or a sintered glass ceramic material made of silicon dioxide.

The transducer body may, for example, be manufactured using 3D printing. It is advantageous to use an additional sintering process after 3D printing to form the transducer body.

In the measuring device of the catheter device, a force effect is measured between transducer portions, which are connected to each other in pairs via a connecting portion and may be moved relative to each other in a predetermined way via the connecting portion. In particular, the transducer portions may, for example, be swivelled and/or axially adjusted in relation to each other so that a force effect along a predefined spatial direction may be determined on the basis of a movement of two transducer portions relative to each other.

The force effect is measured here via a sensor device, which is designed to indicate a deformation between adjacent transducer portions on the basis of a measurement signal, so that, by evaluating the measurement signal, a force value may be determined, which indicates a force acting on the catheter device. The sensor device may be designed as an optical sensor device. The sensor device may, for example, have an optical fibre, in particular in the form of a glass fibre, on which at least one optical device for measuring a force effect between the transducer portions of the at least one pair of adjacent transducer portions is formed. The optical device for measuring a force effect between the transducer portions of the at least one pair of adjacent transducer portions may be formed as an optical grating.

Such an optical grating may, for example, be formed by a Fiber Bragg Grating (FBG), a Long Period Grating (LPG) or a Fabri-Perot resonator, with at least one optical grating being advantageously assigned to each pair of transducer portions, so that measured values may be recorded between each pair of transducer portions in order to evaluate force values on the basis of a deflection between two transducer portions in order to determine a force acting on the catheter device.

In one embodiment, the optical fibre is extended along the longitudinal axis on the transducer body. The optical fibre is firmly connected here to the transducer body at least at defined fastening regions, in particular in the region of the transducer portions, so that a deflection of transducer portions relative to each other leads to a change in length, in particular elongation or compression, at the optical fibre and may thus be measured via the optical gratings of the optical fibre.

In general, a change in length in the form of an elongation or compression at the optical fibre leads to a change in the optical properties of the optical gratings, in particular a change in the grating spacing of the optical gratings. From a change in length at the optical fibre, it may be concluded that there is a force effect at the transducer body and thus at the catheter device, and the force effect may be determined, for example, on the basis of a calibration, within the framework of which, before the actual operation, a deflection at the transducer body and an associated deformation, in particular elongation or compression at the optical fibre, is related to a force effect at the transducer body.

The fact that the transducer body is made of a glass material, especially a quartz glass, a glass ceramic or a sintered glass ceramic, results in advantageous elastic properties at the transducer body. This makes it possible, by means of suitable calibration before the catheter device is put into operation, to carry out measurements of forces acting on the catheter device during operation in a manner that is stable in the long term, since a deflection of transducer portions of the transducer body in relation to each other when force is applied, for example distally on the catheter device, results in the generation of measured values which may be evaluated for determining the force.

If, in addition, the optical fibre of the sensor device is designed as a glass fibre, there is the possibility of an advantageous connection of the optical fibre with the transducer body made of glass material. For example, the glass fibre may be connected to transducer portions of the transducer body by bonding, for example using glass frit, by glass soldering or by welding. Alternatively, for example, a form fit may be produced, for example by thermal clamping between the optical fibre and the transducer portions of the transducer body, in order to connect the optical fibre to the transducer body. Again, as an alternative, the optical fibre, when producing the transducer body, may be connected to the transducer body by sintering in a sintering process. Another possibility to connect the optical fibre to the transducer body is to melt small prepared glass bridges coloured by laser pulses, the glass bridges turning into melt drops and enclosing the glass fibre, cooling down rapidly at the same time and thus creating the fixation. Another possibility to connect the optical fibre to the transducer body is to coat the glass fibre with a suitable metal thin film. The transducer body may be provided with a further suitable layer (or a layered composite), so that the optical fibre may be connected to the transducer body by a solder, for example a eutectic gold-tin (AuSn) solder. This joining technique is also called eutectic soldering. This type of connection, which may be carried out in particular without the use of adhesive, may prevent signal artefacts that could be caused by elastoplastic or tribological effects or degradation of the connection over the service life of the catheter device.

As the transducer body is made of glass material, the measuring device is MR-compatible without further measures and may also be combined with magnetic field sensors, for example for the purpose of catheter navigation.

In one embodiment, the catheter device has a pigtail connected to the transducer body and separate from the optical fibre, which pigtail is optically coupled to the optical fibre at a connection point on the transducer body. The fact that the pigtail is designed separately from the optical fibre, i.e. not in one piece with the optical fibre, and is connected to the optical fibre by optical coupling, prevents mechanical influences on the pigtail, for example tribological influences, from influencing sensor signals. In particular, any movement or force effect on the pigtail is not directly transmitted to the optical fibre of the sensor device and thus cannot lead to artefacts in sensor signals. In addition, the use of different fibres for the optical fibre of the sensor device on the one hand and for the pigtail as the feed line for the optical fibre of the sensor device on the other hand makes it possible to adapt the fibres to the particular requirements, for example by using fibres with different diameters. The pigtail should be sufficiently stable to prevent damage, especially breakage during operation, so that the pigtail should have a larger diameter, for example. By contrast, the optical fibre of the sensor device should be sufficiently elastically extensible to provide a favourable sensitivity for force measurement. The optical fibre of the sensor device may therefore have, for example, a smaller (outer) diameter than the pigtail. The use of different fibres for the optical fibre of the sensor device on the one hand and for the pigtail as the supply line for the optical fibre of the sensor device on the other hand also enables the sensor device to be produced in a pre-assembled and manageable form and thus to be transported more safely (for example to avoid a breakage of the optical fibre) to another location for final assembly in a catheter.

In one embodiment, the connection point on the transducer body is designed in such a way that it aligns the optical fibre and the pigtail centrally in relation to each other. This maximises the transmitted optical sensor signal.

In a further embodiment, the connection point where the optical fibre is optically coupled to the pigtail is provided with an adhesive to adjust the refractive indices of the optical fibre and the pigtail.

In one embodiment, the transducer portions of the transducer body are arranged in a line along a longitudinal axis. The longitudinal axis of the transducer body corresponds advantageously to the longitudinal axis of the catheter device, so that the transducer portions are connected longitudinally to each other on the catheter device, with adjacent transducer portions, in pairs, each being connected to each other via a connecting portion that may be deformed in a defined manner.

By means of the connecting portions, the transducer portions may be moved relative to each other in different ways so that a force effect may be measured via the sensor device assigned to the pairs of transducer portions. The transducer body splits a force applied to the catheter device into vector components, which may be measured by different optical gratings of the optical fibre of the sensor device.

In one embodiment, the transducer body has, for example, at least one pair of adjacent transducer portions which are connected to each other by a connecting portion which is movable around a defined spatial direction extending transversely to the longitudinal axis. The transducer portions are thus, by specifying the connecting portion, movable relative to each other about the spatial direction defined by the connecting portion, so that a force effect about the particular spatial direction may be detected on the basis of a force measurement using an optical grating assigned to the particular pair of transducer portions.

The transducer portions of a pair of adjacent transducer portions are fundamentally only movable around the defined spatial direction, so that a deflection at the transducer portions transverse to the longitudinal axis and transverse to the defined spatial direction may be measured on the basis of the sensor device.

In this case, the connecting portion may, for example, be designed as a web extending between the assigned adjacent transducer portions, which web may be moved flexibly, substantially exclusively around the assigned, defined spatial direction.

In an advantageous embodiment, there may be at least two pairs of adjacent transducer portions, each connected to the other by a connecting portion, the connecting portions being movable about different defined spatial directions extending transversely to the longitudinal axis and thus defining a movability of the transducer portions about different spatial directions. The different spatial directions may, for example, be directed perpendicularly to each other and thus define a plane of movement perpendicular to the longitudinal axis of the transducer body, so that a force effect in the plane of movement extending perpendicularly to the longitudinal axis may be determined via the optical gratings assigned to the pairs of transducer portions.

For example, two pairs of transducer portions may be used to measure a radial force effect on the catheter device along radially directed spatial directions offset by 90° to each other. By contrast, a further, third pair of transducer portions may be used to receive and measure an axial force along the longitudinal axis of the catheter device, so that a force effect may be measured in three-dimensional space using the three pairs of transducer portions.

The third pair of transducer portions is also connected to each other via an associated connecting portion, the connecting portion being shaped in such a way that, when axial force is applied to the catheter device, the transducer portions are deflected relative to each other and thus an axial force is preferably received and measured at this pair of transducer portions.

In one embodiment, the transducer body has a cooling channel for conducting a coolant. The cooling channel is, for example, extended longitudinally along the longitudinal axis through the transducer body, and the cooling channel may be designed concentrically with a cylinder axis of the transducer body, which is cylindrical in its basic form, or also may be designed eccentrically to the cylinder axis. In particular, in order to form recesses in the transducer body to separate the transducer portions from each other, an eccentric arrangement of the cooling channel in the transducer body may be advantageous in order to allow one or more recesses to extend beyond the cylinder axis.

During operation a coolant may be passed through the cooling channel. The coolant, for example in the form of a temperature-controlled physiological saline solution, may also be used for flushing and may be conducted through the transducer body towards the distal end of the catheter device, in particular to a functional electrode of the catheter device arranged at the distal end, in order to exit the catheter device at the functional electrode.

Due to the fact that the transducer body is temperature-controlled by the coolant during operation of the catheter device, temperature-induced artefacts in the force measurement may be reduced. By keeping the transducer body at a substantially constant temperature when the catheter device is in use, a temperature-induced change in length at the optical fibre of the measuring device may be avoided, and therefore it may be assumed that measurement signals obtained using the optical gratings are at least approximately not influenced by temperature effects. This makes it possible to dispense with additional temperature compensation for the force measurement.

Because the transducer body is made of a glass material, in particular quartz glass, biocompatibility is achieved, although the coolant passed through the transducer body may also be used for flushing in the region of the functional electrode, and the transducer body may therefore be in indirect contact with the patient's tissue and blood circulation.

The cooling channel may be straight and axially extended through the transducer body. It is also conceivable, however, that the cooling channel is, for example, meander-shaped and/or designed with a lumen of varying internal diameter in the transducer body. In this way, a convective heat exchange of the coolant with the transducer body may be improved, with a laminar flow within the cooling channel, which may have a heat-insulating effect, being reduced.

In one embodiment, the catheter device has a substantially rigid protective sleeve, in which the transducer body is arranged and to which the transducer body is connected. The protective sleeve encloses the transducer body towards the outside and thus provides a sleeve for the transducer body. The protective sleeve may, for example, have a cylindrical shape which accommodates the transducer body inside. The protective sleeve may, for example, be made of a metal material or a fibre-reinforced plastics material, for example a glass- or carbon-fibre-reinforced plastics material, or a non-fibre-reinforced hard plastics material, and is substantially rigid, i.e. it is not elastically deformable under forces acting during operation.

The transducer body is preferably firmly connected to the protective sleeve at one end, but otherwise extends freely inside the protective sleeve. For example, there may be a (air) gap between the transducer body and the protective sleeve where the transducer body is not connected to the protective sleeve, so that the transducer body is arranged and received with play in the protective sleeve and may thus move in the protective sleeve under elastic deformation.

Via the (air) gap, the transducer body is preferably thermally insulated from the protective sleeve so that temperature control of the transducer body is supported by a coolant passing through the transducer body.

For example, the transducer body may be connected to the protective sleeve at a first end and may be movable relative to the protective sleeve with a second end remote from the first end, so that the transducer body may be deflected with the second end in the protective sleeve, in particular radially. At the first end, the transducer body may be fixed to the protective sleeve in the manner of a cantilever beam, for example, so that the transducer body is fixed at the first end in relation to the protective sleeve in terms of position and torque. A deflection of the second end is thus effected with elastic deformation of the transducer body, in particular with deflection of the transducer portions relative to each other.

In one embodiment, the catheter device has a functional electrode which - when the catheter device is designed as an ablation catheter - serves, for example, to feed electrical energy into the tissue to be treated and is positioned distally on the catheter device for this purpose. The functional electrode is connected, for example, to the second end of the transducer body, so that a force acting on the functional electrode as a result of contact of the catheter device with surrounding tissue leads to elastic deformation and deflection at the transducer body and thus a force acting on the functional electrode may be measured on the basis of the deformation of the transducer body. Typically, the force effects to be measured are in the range of 0.05 N to 0.5 N, preferably in the range of 0.2 N to 0.4 N. If the catheter device is inserted into the body or removed from the body via the vascular system, a force effect exceeding 0.5 N may occur in the region of the force measuring device and in particular in the region of the transducer body. Force effects in the region of the transducer body of up to 20 N are not unusual when inserting and removing the catheter device, especially if the insertion and removal is carried out through an insertion catheter. In this case it is helpful if the force measuring device, especially the transducer body, is protected by a protective sleeve, i.e. the deformation of the transducer body is limited by the protective sleeve. Typical values for the force effect with which this protective effect should take effect are in the range of 0.5 N to 2 N.

The functional electrode may, for example, form a distal termination of the catheter device and is exposed outwardly during operation of the catheter device. At its distal end, the functional electrode may in particular have a hemispherical shape.

In one embodiment, the catheter device has one or more electrode poles, which serve, for example, to sense electrical signals from tissue and are arranged distally on the catheter device for this purpose. These electrode poles may be designed as ring electrodes, which may be used for diagnostic mapping, for example. The ring electrodes are preferably arranged on the protective sleeve.

In one embodiment, the catheter device has an elastically deformable sealing element to seal a transition between the protective sleeve and the second end of the transducer body. The sealing element is flexibly deformable and is designed to seal the transition between the protective sleeve and the transducer body, even in the event of a deflection at the transducer body, in such a way that no liquid may penetrate the protective sleeve.

The sealing element may, for example, be made of an elastomeric plastic and is flexibly deformable.

In one embodiment, the protective sleeve forms a stop to limit elastic deformation of the transducer body. The stop may be formed especially in the region of the second end of the transducer body, so that a radial deflection of the transducer body may be limited and an overstressing of the transducer body due to an overload and a resulting deformation of the transducer body may be avoided. The stop may, for example, be formed by a circumferential edge at the distal end of the protective sleeve, so that the stop limits deflection of the transducer body in all radial directions.

In one embodiment, the catheter device has an evaluation device which is designed to evaluate an optical signal to determine a force effect on the transducer body. The optical signal is obtained as a response signal to a fed-in optical signal and may be present in particular as a reflection signal which has been reflected at the optical gratings of the sensor device. The optical signal may be evaluated in order to determine a force effect on the transducer body on the basis of the optical signal, in particular on the basis of a change in the optical signal compared to a reference signal in a defined, unloaded initial state of the transducer body.

The optical gratings may be arranged on a single optical fibre. An evaluation may be carried out here using a single optical signal, in particular one fed-in in broadband, with the optical gratings differing in their optical properties and reflecting in particular in the range of different wavelengths. Optical signals obtained from different gratings therefore differ in wavelength and may be differentiated and evaluated accordingly in order to evaluate information from the individual gratings and thus determine a force effect in the region of the different gratings and thus along the different force directions assigned to the different gratings.

The evaluation device may, in one embodiment, be designed in particular to evaluate a wavelength change of the optical signal to determine the force effect. When a force is applied to the transducer body and two transducer portions are thus deflected relative to each other, this causes a change in the length of the optical fibre in the region of the optical gratings assigned to the transducer portions, in particular an elongation or compression of the optical fibre and thus a change in the optical properties of the optical gratings of the sensor device. In particular, a grating period of the optical gratings may change, which leads to a change in the received reflection signal and may be evaluated accordingly.

The idea underlying the invention will be explained in more detail below on the basis of the exemplary embodiments shown in the figures, in which:

Fig. 1 shows a view of an embodiment of a catheter device with a measuring device;

Fig. 2 shows a view of a distal portion of the catheter device with the measuring device arranged thereon;

Fig. 3A shows a longitudinal sectional view through the measuring device at the distal portion of the catheter device, with a cooling channel in a transducer body of the measuring device;

Fig. 3B shows a sectional view of a transducer body with a cooling channel according to another embodiment;

Fig. 4A shows a cross-sectional view through the transducer body in the region of a connecting portion according to Fig. 3A in the region of a first optical grating of an optical fibre;

Fig. 4B shows a cross-sectional view through the transducer body in the region of a connecting portion according to Fig. 3A in the region of a second optical grating of the optical fibre;

Fig. 4C shows a cross-sectional view through the transducer body in the region of a connecting portion according to Fig. 3A in the region of a third optical grating of the optical fibre;

Fig. 5 shows a cross-sectional view of the transducer body in the region of a transducer portion between two optical gratings;

Fig. 6 shows a cut-open view of the catheter device, showing the transducer body;

Fig. 7 shows the view according to Fig. 6, with a deflection of the transducer body;

Fig. 8 shows a view of a functional electrode at the distal end of the catheter device;

Fig. 9 shows a Cartesian signal diagram of recorded measurement signals;

Fig. 10 shows half of the Cartesian signal diagram according to Fig. 9 assigned to pressure forces;

Fig. 11 shows a two-dimensional characteristic diagram of the measuring device;

Fig. 12 shows a view of the transducer body at a connection point for connecting a pigtail; and

Fig. 13 shows an enlarged longitudinal sectional view in the region of the connection point.

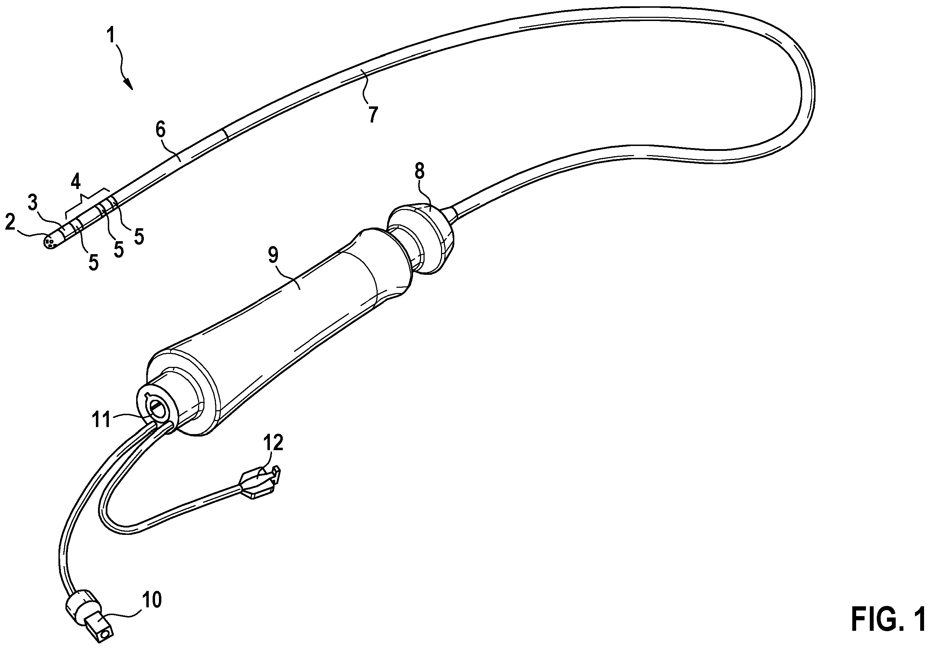

Fig. 1 shows a schematic view of a catheter device 1, which may, for example, form an ablation catheter and has a measuring device for measuring a contact force.

In the embodiment shown in Fig. 1, the measuring device is designed to improve the quality of therapeutic lesion formation by providing real-time information regarding the contact force of a functional electrode 2 with regard to direction and strength on a target tissue 13 during ablation. Furthermore, the real-time information regarding the contact force may be used to improve patient safety during catheter positioning in order to avoid vascular and cardiac wall perforations caused by excessive forces or unfavourable orientation of the functional electrode 2.

Fig. 1 shows the catheter device 1 with all its components. Peripheral devices for generating ablation energy and for recording, evaluating and displaying electrical and optical signals are not shown for the sake of clarity.

The catheter device 1 has a flushed functional electrode 2 in the form of a head electrode, which is designed for performing an ablation and is arranged distally of an elastic sealing element 3. The sealing element 3 is designed to allow elastic movability of the functional electrode 2 relative to a rigid segment 4 of the catheter device 1 and to seal a transition between the functional electrode 2 and the rigid segment 4 in a fluid-tight manner so that liquid from the outside cannot penetrate into the interior of the catheter device 1. The sealing element 3 is firmly connected to the transducer body 15 on the one hand and the rigid segment 4 on the other hand.

The rigid segment 4 serves to surround a transducer body 15 of the measuring device, which is enclosed in rigid segment 4, outwardly and thus protect it against external mechanical stress and thermal influences. A number of ring electrodes 5 are arranged on the rigid segment 4, in the embodiment shown three ring electrodes 5, and may be used for diagnostic mapping, for example.

A controllable segment 6 is connected proximally to the rigid segment 4. The controllable segment 6, which may be deflected by bending a control button 8 on a handle 9 to deflect and control the functional electrode 2, is connected to a catheter shaft 7, which is flexible, but torsion- and compression-resistant and cannot be controlled.

Optical measuring signals generated using the measuring device may be fed via an optical connection 10 in the form of a glass fibre connection on the handle 9, for example, to an external control and evaluation device.

An electrical connection 11 is also located on the handle 9, via which electrical connection ablation energy may be introduced into the functional electrode 2, and electrical signals, which are obtained via the ring electrodes 5, for example, may be diverted. A flushing connection 12 in the form of a Luer lock closure is provided to introduce a cooling and flushing liquid, for example a physiological saline solution, for the functional electrode 2 and the measuring device on the handle 9.

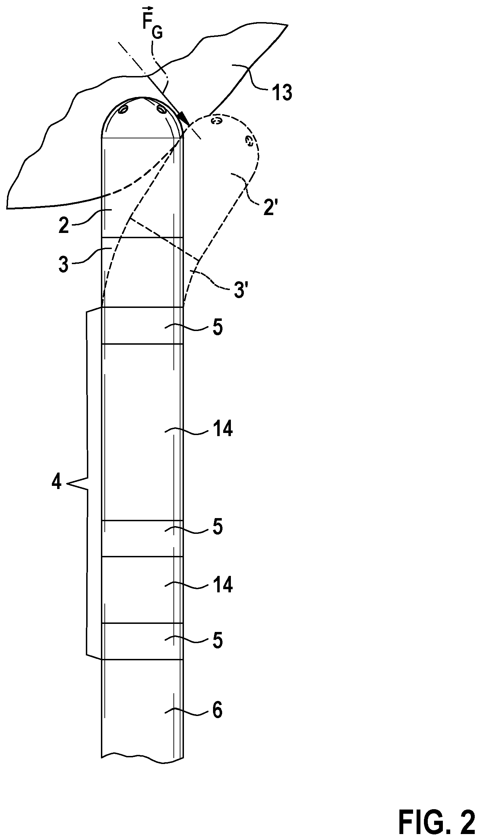

As may be seen in Fig. 2, the rigid segment 4 has an insulating layer 14, formed for example from an LCP ("liquid crystal polymer"). The insulating layer 14 forms an outer peripheral surface of the rigid segment 4, on which the ring electrodes 5 are formed, for example by copper etching and gold deposition. Electrical connecting lines of the ring electrodes 5 run within the insulation layer 14.

The elastic sealing element 3 enables the functional electrode 2 to move relative to the rigid segment 4 when it comes into contact with tissue 13 (illustrated by the dashed functional electrode 2' in Fig. 2), since the sealing element 3 may deform elastically when subjected to a load on the functional electrode 2 (illustrated by the deformed sealing element 3' in Fig. 2). As will be explained below, a load acting on a contact plane between the functional electrode 2 and tissue 13 may be influenced in its effect on the functional electrode 2 by a resulting force vector $\overrightarrow{F_G}$ with regard to amount and direction.

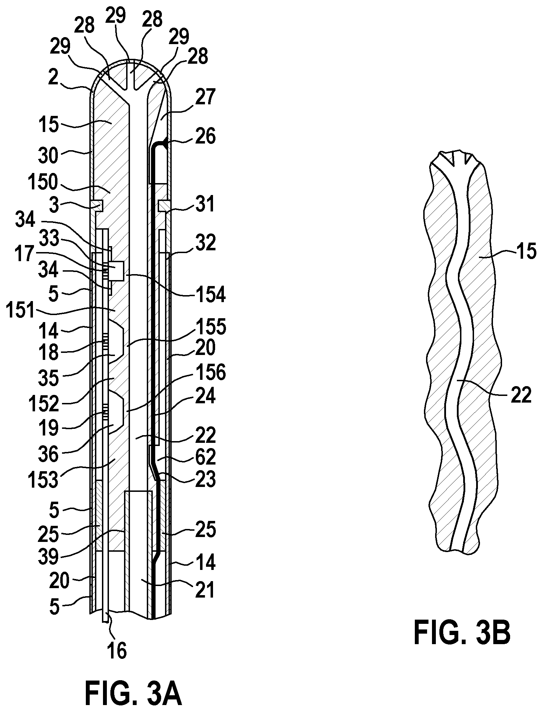

Fig. 3A shows a longitudinal section through a distal portion of the catheter device 1 extended along a longitudinal axis L.

As may be seen in Fig. 3A, the measuring device has a transducer body 15 made of glass material, an optical fibre in the form of a glass fibre 16 with internal optical gratings 17, 18, 19, for example in the form of so-called Fiber Bragg Gratings or Long-Period Bragg Gratings, and a protective sleeve 20.

The transducer body 15 is manufactured in one embodiment from a glass material, preferably a high-purity quartz glass, in 3D printing with a subsequent sintering process.

In an alternative embodiment, the transducer body 15 is made of a glass material, for example a glass ceramic material or a sintered glass ceramic material.

The glass fibre 16, for example, has a light-conducting core with a diameter between 5 $\mu$m and 15 $\mu$m, for example 9 $\mu$m, and a glass sheath with a diameter between 80 $\mu$m and 200 $\mu$m, for example 125 $\mu$m. The glass sheath may be surrounded by an insulating polyamide coating, so that the glass fibre 16, for example, has a total diameter between 100 $\mu$m and 250 $\mu$m, for example 155 $\mu$m. During operation, light with a short coherence length is coupled into the light-conducting core via the optical connection 10 (see Fig. 1) and reflected at the optical gratings 17, 18, 19 in distinguishable light wavelength ranges. The reflected wavelength signals are converted into measurement signals S1, S2, S3 and evaluated by an external device connected to the optical connection 10, for example in the form of a so-called interrogator.

The fact that the transducer body 15 is made of a glass material, in particular a quartz glass, a glass ceramic or a sintered glass ceramic, results in an advantageous connection with the glass fibre 16. For example, the glass sheath of the glass fibre 16 may be exposed for fixing, for example, and fused, welded, soldered or inseparably connected by (anodic) bonding to the quartz glass, the glass ceramic or the sintered glass ceramic of the transducer body 15. By such a material connection of the transducer body 15 to the glass sheath of the glass fibre 16, hysteresis-effective elastoplastic couplings of a force-induced or thermal deformation of the transducer body 15 into the glass fibre 16 and thus into its optical gratings 17, 18, 19, which could otherwise occur, for example, in adhesive bonding, may be avoided. In addition, by using the glass sheath of the glass fibre 16 with the transducer body 15 consisting of glass or glass ceramic, a long-term and age-resistant connection may be created, without the connection being impaired over the service life of the measuring device.

The transducer body 15 has transducer portions 150-153 lined up along the longitudinal axis L and connected in pairs by connecting portions 154-156 and decoupled from each other by recesses 33-36 in such a way that the transducer portions 150-153 may be deflected elastically in pairs, as will be explained below. The glass fibre 16 is connected to the transducer portions 150-153 of the transducer body 15 in such a way that regions of the glass fibre 16 where the optical gratings 17, 18, 19 are located are freely extended over the recesses 33, 34, 35, 36.

At the proximal end of the transducer body 15, a flushing tube 21 for cooling the tissue 13 during ablation via the functional electrode 2 is glued into a flange 39 formed on the transducer body 15. This has the advantage that a flushing liquid, for example in the form of a physiological saline solution, may be led via the flushing connection 12 through a cooling channel 22 in the transducer body 15, and thus the transducer body 15 and thus also the glass fibre 16 may be cooled and kept at a constant temperature. For example, a material bond between the glass sheath of the glass fibre 16 and the transducer body 15 results in a favourable heat transfer between the glass fibre 16 and the transducer body 15. A temperature-induced drift of the measuring signals S1, S2, S3 due to thermal expansion of the optical gratings 17, 18, 19 may thus be counteracted. It may be that a thermosensor for temperature correction of the measured values is not necessary.

When using the catheter device 1 to perform an ablation, the inner surface of the cooling channel 22 is in indirect blood contact via the flushing fluid. Because the inner surface of the transducer body 15 which limits the cooling channel 22 is made of a glass material, preferably a high-purity quartz glass, glass ceramic or sintered glass ceramic, corresponding to the material of the transducer body 15, biocompatibility is guaranteed for temporary use in a catheter in the patient.

The arrangement has the further advantage that there are no tribological interactions of the transducer body 15 with a flushing line led to the functional electrode 2, and thus no signal shifts or signal artefacts such as jumps due to tribological interactions may occur.

The transducer body 15 is connected to the protective sleeve 20 proximally at an axial location 25. Distally, the transducer body 15 and the protective sleeve 20 are connected to each other via the elastic sealing element 3. The sealing element 3 is connected interlockingly at an axial location 32 to the protective sleeve 20 and at a location 31 to the transducer body 15.

The connection at the locations 25, 31, 32 is preferably linear along peripheral connecting lines.

The protective sleeve 20 limits the deformation of the transducer body 15 and thus of its optical gratings 17, 18, 19, which is necessary for measuring the contact force. The transducer body 15 is thus prevented from breaking due to overload, for example when the catheter 1 passes through haemostasis valves or curved sluice catheters.

In addition, the outer surface of the protective sleeve 20 has an insulating layer 14 (for example made of LCP) on which the ring electrodes 5 are attached. The axial extent of the rigid segment 4 is determined substantially by the length of the protective sleeve 20.

The sealing element 3 enables the deformation of the transducer body 15 and thus of its optical gratings 17, 18, 19, which is necessary for measuring the contact force, and prevents the penetration of body fluid.

The functional electrode 2 is adhesively fixed at an axial location 30 on the transducer body 15 in such a way that the flushing openings 29 of the functional electrode 2 coincide with the branched flushing channels 28 connected to the flushing channel 22.

The transducer body 15 has a cable channel 24, through which an electrical cable 23 extends to the functional electrode 2. The electrical cable 23 is connected to the functional electrode 2 at a soldering point 26 in the region of a recess 27, which facilitates the mounting of the functional electrode 2 on the transducer body 15 with soldered cable 23, as shown in Fig. 3A.

Fig. 3B shows a detail of an embodiment of the transducer body 15, which, compared to the embodiment according to Fig. 3A, has a meandering cooling channel 22, in which the formation of a laminar flow with possibly reduced heat transfer capacity may at least be reduced.

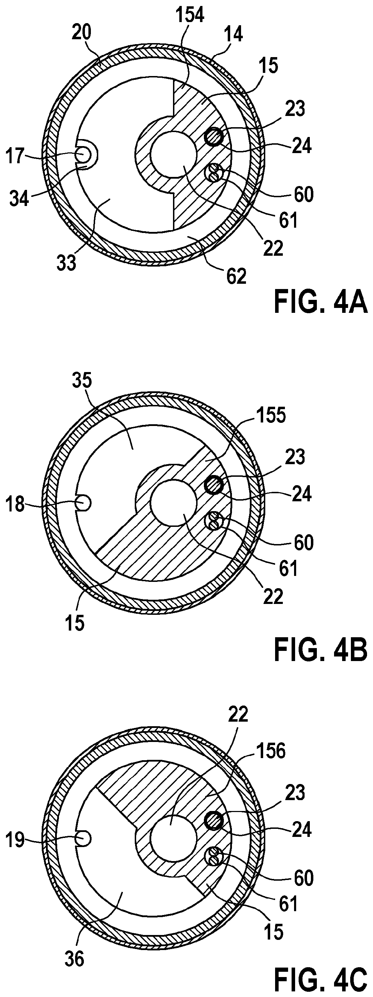

Figs. 4A, 4B and 4C show axially offset cross-sections through the connecting portions of the distal section of the catheter device 1.

For example, Fig. 4A shows a sectional view of the measuring device in the region of the optical grating 17 and the associated recess 33 which decouples the transducer portions 150, 151 from each other. As may be seen in Fig. 4A in conjunction with Fig. 3A, the recess 33 has a comparatively short axial length but a comparatively large radial depth extending beyond the centre of the transducer body 15, as may be seen in particular from the sectional view according to Fig. 4A. The recess 33 is axially connected to a channel 34 on each side, in which channel the glass fibre 16 is freely extended in such a way that in the region of the channels 34 the glass fibre 16 does not contact the transducer body 15.

Parallel to the cable channel 24, a channel 61 extends through the transducer body 15, in which a cable for a thermocouple 60 may be laid.

In the embodiment shown, the cooling channel 22 is arranged eccentrically to the centre of the transducer body 15, which has a basic cylindrical shape, in order to create space for the recesses 33, 35, 36.

Between the protective sleeve 20 and the transducer body 15 there is an air gap 62, which serves as thermal insulation outwardly and thus supports the cooling effect on the optical gratings 17, 18, 19 via the cooling channel 22. In addition, the air gap 62 allows a deformation of the transducer body 15 and thus a sectional movement of the transducer body 15 within the rigid segment 4.

Because the recess 33 extends into the transducer body 15 over a comparatively small axial length but a comparatively large radial depth, the connecting portion 154 forms a connection in the manner of a short bar (corresponding to the connecting portion 154 of transducer body 15, shown hatched in Fig. 4A) for connecting the transducer portions 150, 151 of the transducer body 15 axially adjacent to the recess 33. Forces acting axially on the transducer body 15 may lead to a deflection of the transducer portions 150, 151 of the transducer body 15 axially adjacent to the recess 33, so that in particular axially acting forces may be detected by the optical grating 17 assigned to the recess 33 and detected by means of an expansion or compression at the optical grating 17.

Fig. 4B shows a sectional view of the measuring device in the region of the optical grating 18 and the associated recess 35 in the transducer body 15. Compared to the recess 33 according to Fig. 4A, the recess 35 has a smaller cross-sectional expansion, but, as may be seen in Fig. 3A, it extends over a greater axial length in the transducer body 15. The connecting portion 155 of the transducer body 15, which connects the transducer portions 151, 152 of the transducer body 15 axially adjacent to the recess 35, thus has a larger cross-sectional region and creates a connection in the manner of an axially comparatively long beam (shown hatched in Fig. 4B). The transducer portions 151, 152 of the transducer body 15, which are axially adjacent to the recess 35, may thus be deflected relative to each other, especially in bending, in accordance with the alignment of the recesses 35, so that forces acting in particular radially on the optical grating 18 may be absorbed by means of an expansion or compression of the optical grating 18.

Fig. 4C shows a sectional view of the measuring device in the region of the optical grating 19 and the associated recess 36, which decouples the transducer portions 152, 153 from each other. The recess 36 is shaped like the recess 35 associated with the optical grating 18, but is rotated by 90° around the longitudinal axis of the transducer body 15 relative to the recess 35 and is thus aligned at right angles to the recess 35. The transducer portions 152, 153 of the transducer body 15 adjacent to the recess 36 may, as for the recess 35, in particular be deflected relative to each other in bending, according to the alignment of the recesses 36, so that forces acting radially on the optical grating 19 in particular may be absorbed by means of expansion or compression of the optical grating 19.

Because the recess 36 is arranged at a right angle to the recess 35, radial forces in particular, which are directed perpendicularly to the radial forces detectable at the optical grating 18, may be received and detected at the optical grating 19.

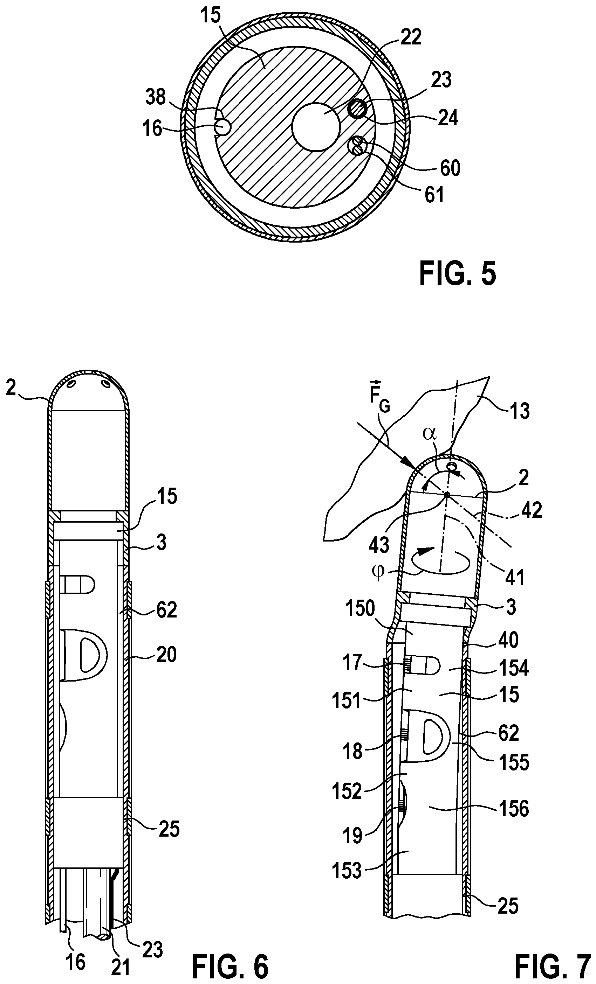

Fig. 5 shows a cross-section of the measuring device in a region of the transducer body 15 between two adjacent recesses 33, 35, 36. The glass fibre 16 is embedded in a groove-shaped channel 38 on the outside of the transducer body 15 and is firmly connected to the transducer body 15 in the region of the channel 38. The glass fibre 16 is firmly connected to the transducer body 15, especially in those portions where it is not freely extended in the region of a recess 33, 35, 36.

The glass fibre 16 has, in one embodiment, a non-polymer-based coating (also referred to as a coating), but may be used in other embodiments without such a coating.

In order to make a connection to the transducer body 15, the glass fibre 16 may, for example, have a coating of a gold material which makes it possible to form a connection of the glass fibre 16 with a metal coating of the transducer body 15 by soldering. In another embodiment, a glass fibre 16 may be bonded to the transducer body 16 without coating by means of glass frit / glass solder. In another embodiment, a transducer body 15 may be fitted with a glass fibre 16 as a so-called green body, i.e. as a blank during production in a sintering process, in order to create a connection between the transducer body 15 and the glass fibre 16 during subsequent sintering. The glass fibre 16 may be connected positively to the transducer body 15 in a different embodiment, for example by thermally expanding the transducer body 15 for the glass fibre 16, attaching the glass fibre 16 to the expanded transducer body 15 and thereby creating a tension between the transducer body 15 and the glass fibre 16 when the transducer body 15 cools down. In another embodiment, the fixing is done by laser pulses on coloured glass bridges, which transform into melt droplets and enclose the glass fibre and cool down rapidly. In another embodiment, one or more optical fibres may be created in the transducer body 15, for example by doping, by using glass materials with different refractive indices or by laser treatment, in order to create a measuring device without a separate glass fibre connected to the transducer body 15.

In Fig. 6 the measuring device at the distal end of the catheter device 1 is shown in partially cut-open view. In particular, Fig. 6 shows the arrangement of the transducer body 15 inside the protective sleeve 20 with the air gap 62 in between. At a first end corresponding to the axial location 25, the transducer body 15 is connected to the protective sleeve 20. At a second end remote from this, the transducer body 15 may move freely in relation to the protective sleeve 20 and engages in the sealing element 3, so that the transducer body 15 may be deflected elastically in relation to the protective sleeve 20 together with the functional electrode 2.

While Fig. 6 shows the measuring device with non-deflected functional electrode 2, Fig. 7 shows the measuring device with force acting on the functional electrode 2 and correspondingly deformed transducer body 15.

As may be seen from Fig. 7, when force is applied to the functional electrode 2, according to the force vector $\vec{F}_G$ at the functional electrode 2, the transducer body 15 is elastically deformed within the protective sleeve 20 and moves within the protective sleeve 20 by deflecting the portions of the transducer body 15 separated from each other by the recesses 33, 35, 36 relative to each other.

While the transducer body 15 is firmly connected to the protective sleeve 20 at the axial location 25 in the manner of a rigid beam clamp, the end of the transducer body 15 assigned to the sealing element 3 may move freely within the protective sleeve 20. Accordingly, the transducer body 15 may be axially lengthened or shortened when force is applied to the functional electrode 2 and may bend in any radial direction, with elastic deformation of the sealing element 3. The elastic sealing element 3 ensures that no body fluid may penetrate into the air gap 62 and a transition between the functional electrode 2 and the protective sleeve 20 is thus sealed fluid-tight.

Due to the fixed connection of the glass fibre 16 to the transducer body 15, the three optical gratings 17, 18, 19 are immediately compressed or stretched according to the magnitude and direction of the force on the functional electrode 2 in the event of deformation at the transducer body 15, so that a force effect in terms of magnitude and direction may be detected on the basis of measurement signals obtained via the optical gratings 17, 18, 19.

A force measurement may be possible up to an amount of 8 N, for example.

To counteract overloading of the transducer body 15, deflection and deformation of the transducer body 15 is limited by a distal inner edge 40 of the protective sleeve 20. The inner edge 40 thus acts as a limit stop.

Due to the temperature control of the transducer body 15 and thus of the glass fibre 16 by coolant flow through the cooling channel 22, it may be assumed that a mechanical expansion $\varepsilon_1$, $\varepsilon_2$, $\varepsilon_3$ of the optical gratings 17, 18, 19 due to force-induced deformation at the transducer body 15 alone is the cause of a displacement $\delta_{\lambda 1}$, $\delta_{\lambda 2}$, $\delta_{\lambda 3}$ of a reflected wavelength $\lambda_{B1}$, $\lambda_{B2}$, $\lambda_{B3}$ at the optical gratings 17, 18, 19 acting as band stops.

For measurement purposes, a wavelength signal is fed into the optical fibre 16, which is reflected at the optical gratings 17, 18, 19. If the signal is stretched or compressed at one of the optical gratings 17, 18, 19, a shift of the wavelength in the associated reflected signal occurs. Each optical grating 17, 18, 19 acts as a narrow-band bandstop with an associated centre wavelength, with the wavelength ranges of the optical gratings 17, 18, 19 differing from one another and thus signals being reflected at different wavelengths at the optical gratings 17, 18, 19.

For the wavelength shift, the following applies:

$$\delta_{\lambda i} \approx 0.78 \cdot \varepsilon_i \cdot \lambda_{Bi}$$

with

i = 1, 2 or 3.

The distance between the band gaps $\lambda_{Bi}$, for example, lies in a range between 5 nm and 30 nm, for example is 10 nm, to ensure reliable differentiation of the wavelength shifts $\delta_{\lambda i}$, which are two to three orders of magnitude smaller.

Three signals S1, S2, S3 of the optical gratings 17, 18, 19 are generated from the wavelength shifts $\delta_{\lambda 1}$, $\delta_{\lambda 2}$, $\delta_{\lambda 3}$. The following applies here:

$$S_i = 1000 \cdot \delta_{\gamma i}$$

The factor 1000 takes into account the ratio $\delta_{\lambda i}/\lambda_{Bi}$.

In principle, three distinguishable optical signals from LPG or Fabri-Perot resonators may perform the same function in addition to the FBG signals described above. These signals must only be proportional to three independent components of the force vector $\overrightarrow{F_G}$.

In Fig. 7 a functional electrode axis 41 is shown, which points through a centre 43 of the functional electrode 20, which is spherical at the distal end, and is aligned along the axial extent of the functional electrode 2. A line of action 42, along which a force vector $\overrightarrow{F_G}$ due to an interaction of the functional electrode 2 with tissue 13, acts on the functional electrode 2, points through the centre 43 and is directed at an elevation angle $\alpha$ and an azimuth angle $\varphi$ to the functional electrode axis 41.

From the signals S1, S2, S3 the force in magnitude and direction may be determined. In this way each force vector $\overrightarrow{F_G}$ may be assigned a signal vector S accordingly:

$$\overrightarrow{F_G} = \begin{pmatrix} F_G \\ \alpha \\ \varphi \end{pmatrix} \rightarrow \begin{pmatrix} S_1 \\ S_2 \\ S_3 \end{pmatrix} = \vec{S}$$

The signals S1, S2, S3 derived from reflection signals of the optical gratings 17, 18, 19 may be used as components of a signal vector $\vec{S}$ be interpreted in a signal space. The result is a system of equations with three equations and three unknowns, namely the magnitude of the force $F_G$ and the angles $\alpha$, $\varphi$, which may be resolved and a determination of the force vector $\overrightarrow{F_G}$ in respect of amount and direction.

In general, it may be assumed that a risk of perforation on the tissue 13 is greatest when the line of action 42 and the functional electrode axis 41 are at least approximately in alignment with each other. The angle $\alpha$ is then at or near 0°, so that the functional electrode 2 is perpendicular to the surface of the tissue 13. If, on the other hand, the functional electrode 2 with the electrode axis 41 is aligned at least approximately parallel to the tissue 13, the angle $\alpha$ has a value of approximately 90° and there is a comparatively small risk of perforation.

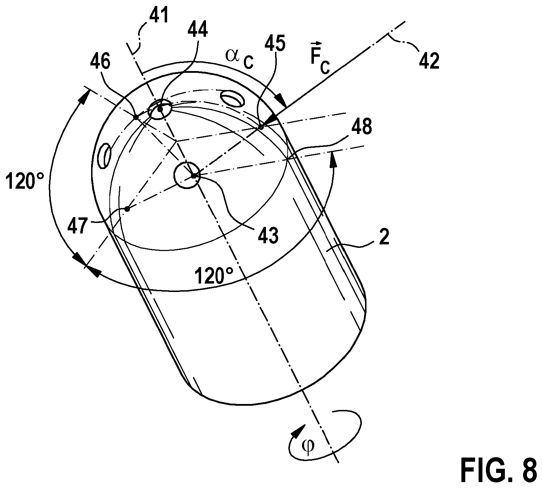

Fig. 8 to 11 are used in the following to explain how the measuring device may be calibrated in order to enable the force vector to be determined under load on the functional electrode 2 during operation.

For calibration, a method for generating a characteristic diagram may be used which establishes a relationship between quantities derived from the measurement signals generated by the measuring device and the magnitude and direction of the force vector.

In particular, calibration may be carried out using a 5-point method, as a result of which calibration values are obtained which are used as parameters in a signal-force determination algorithm. After calibration has been carried out, the magnitude of the force $F_G$ and the angle $\alpha$ may be calculated from the three derived signals S1, S2, S3 of the optical gratings 17, 18, 19 in a control and evaluation device implemented in a peripheral device and connected to the optical connection 10, using the algorithm. The following applies:

$$\vec{S} = \begin{pmatrix} S_1 \\ S_2 \\ S_3 \end{pmatrix} \rightarrow \begin{pmatrix} F_G \\ \alpha \end{pmatrix} = \overrightarrow{F_G}^*$$

The magnitude of the force $F_G$ and the angle $\alpha$, which may be seen as components of a force vector $\overrightarrow{F_G}^*$ may be interpreted on one level, may be displayed digitally on a monitor and thus may be made available to a user as information.

Fig. 8 shows an example of five calibration points 44, 45, 46, 47, 48 on the functional electrode 2. Each calibration point 44, 45, 46, 47, 48 corresponds to a point of action at which a force is exerted on the functional electrode 2 during calibration. During calibration, the amount and direction of the force is known and a signal S1, S2, S3 obtained from a deformation on the optical gratings 17, 18, 19 may be evaluated and calibrated accordingly.

If the functional electrode 2, as shown in the present example, has the shape of a hemisphere at its distal end and the distal end face of the functional electrode 2 thus corresponds to a (hemi-)spherical surface, all lines of action of the resulting forces $F_G$ run (neglecting friction) between the functional electrode 2 and tissue 13 (in Fig. 8 the line of action 42 for the calibration point 45 is shown) through the centre 43 of the spherical surface, corresponding to the intersection of the functional electrode axis 41 and the line of action 42.

If the shape of the functional electrode 2 deviates from a spherical shape, a spherical cap with a defined point of action may be placed on functional electrode 2 (play-free) for calibration.

For calibration, a calibration force $\left|\overrightarrow{F_C}\right| = F_C$ and a calibration angle $\alpha_C$ between 0° and 90° is selected. Values such as those that may occur during later operational use are useful here, for example. For example, a value of $F_C = 0.29\,N$, corresponding to 30 g (this corresponds to a reference weight as it is often used in medical practice), and $\alpha_C = 45°$ may be used for the amount of the calibration force - assuming that the functional electrode 2 will seldom lie exactly perpendicularly or exactly parallel to the surface of a tissue 13 during ablation. In this way it is possible to calibrate to a high measuring accuracy in a working region.

The calibration point 44, to which the calibration force $F_C$ is applied, is, for example, on the functional electrode axis 41 of functional electrode 2.

The calibration points 45, 46, 47 are at an angular distance $\alpha_C$ (for example 45°) from the functional electrode axis 41. The calibration points 45, 46, 47 must be rotated by $\Delta\varphi = 120°$ to each other around the axis of rotation.

The calibration point 48 is at an angular distance $\alpha_C = 90°$ to the functional electrode axis 41 and is shifted by $\Delta\varphi = 0°$ to one of the calibration points 45, 46, 47. (The absolute angle of rotation $\varphi$ of the functional electrode 2 has no significance for calibration).

Each of the calibration measurements at the five calibration points 44, 45, 46, 47, 48 yields a signal vector $\vec{S}$ with three components each. This means that 15 individual values are recorded. Of these, for example, twelve parameters (calibration values) are selected for the signal-force determination algorithm.

From the signal vector $\overrightarrow{S_I}$ of the first calibration point 44, with $F_C = 30$ g and $\alpha_C = 0°$, four parameters may be calculated for the signal-force determination algorithm:

$$\overrightarrow{S_I}(30g, 0°) \rightarrow \vec{b}_{30g,0°} = b_{30g,0°} \cdot \overrightarrow{e_b}$$

From the signal vectors $\overrightarrow{S_{II}}, \overrightarrow{S_{III}}, \overrightarrow{S_{IV}}$ of the calibration points 45, 46, 47, with $F_C = 30$ g and $\alpha_C = 45°$ another seven parameters may be calculated for the signal-force determination algorithm:

$$\overrightarrow{S_{II}}(30g, 45°, \varphi), \overrightarrow{S_{III}}(30g, 45°, \varphi + 120°), \overrightarrow{S_{IV}}(30g, 45°, \varphi + 240°)$$

$$\rightarrow \vec{b}_{30g,45°}, \overrightarrow{f1}_{30g,45°}, \overrightarrow{f2}_{30g,45°}$$

$\vec{b}_{30g,45°}$ is discarded in favour of $\vec{b}_{30g,0°} = b_{30g,0°} \cdot \overrightarrow{e_b}$ (see above). From $\overrightarrow{f1}_{30g,45°}$ and $\overrightarrow{f2}_{30g,45°}$ only $\overrightarrow{e_{f1}}$, $\overrightarrow{e_{f2}}$ and $c = \dfrac{|\overrightarrow{f1}|}{|\overrightarrow{f2}|}$ are adopted.

From the signal vector $\overrightarrow{S_V}$ of the calibration point 48, with $F_C = 30$ g and $\alpha_C = 90°$ for $\phi$, $\varphi+120°$ or for $\varphi+240°$, the still missing parameter for the signal-force determination algorithm may be calculated:

$$\overrightarrow{S_V}(30g, 90°, \varphi) \rightarrow \overrightarrow{f1}_{30g,90°}$$

All that is required is $|\overrightarrow{f1}_{30g,90°}| = f1_{30g,90°}$

This results in twelve calibration parameters (calibration values):

$$\begin{pmatrix} e_{f11} \\ e_{f12} \\ e_{f13} \end{pmatrix}, \begin{pmatrix} e_{f21} \\ e_{f22} \\ e_{f23} \end{pmatrix}, \begin{pmatrix} e_{b1} \\ e_{b2} \\ e_{b3} \end{pmatrix}, f1_{30g,90°}, b_{30g,0°}, c$$

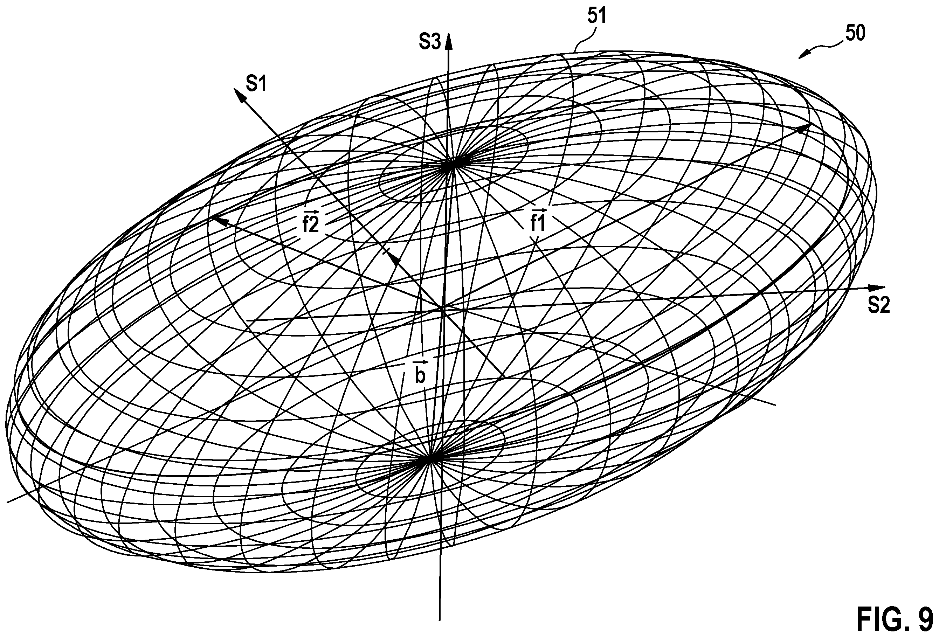

Fig. 9 shows a Cartesian signal diagram 50, whose axes represent the values of the derived signals S1, S2, S3 from the reflected wavelengths of the respective optical gratings 17, 18, 19.

The diagram shows an isoforce area 51 as a grid. All signal vectors $\vec{S}$ which end from the zero point on this isoforce area 51 are determined by a force vector $\overrightarrow{F_G}$ of the same amount $F_G$ is generated. The isoforce area 51 has the shape of a tri-axial ellipsoid, where the vectors $\overrightarrow{e_{f1}}$, and $\overrightarrow{e_{f2}}$ are orthogonal to each other. The vector $\overrightarrow{e_b}$, may be arbitrary, but must be linearly independent of $\overrightarrow{e_{f1}}$ and $\overrightarrow{e_{f2}}$ be.

For $F_C = 30$ g, the isoforce area 51 is fully described with the following vectors shown in Fig. 9 and also includes possible tensile forces:

$$\overrightarrow{f1} = f1_{30g,90°} \cdot \begin{pmatrix} e_{f11} \\ e_{f12} \\ e_{f13} \end{pmatrix},$$

$$\overrightarrow{f2} = \frac{f1_{30g,90°}}{c} \cdot \begin{pmatrix} e_{f21} \\ e_{f22} \\ e_{f23} \end{pmatrix}$$

and

$$\vec{b} = b_{30g,0°} \cdot \begin{pmatrix} e_{b1} \\ e_{b2} \\ e_{b3} \end{pmatrix}$$

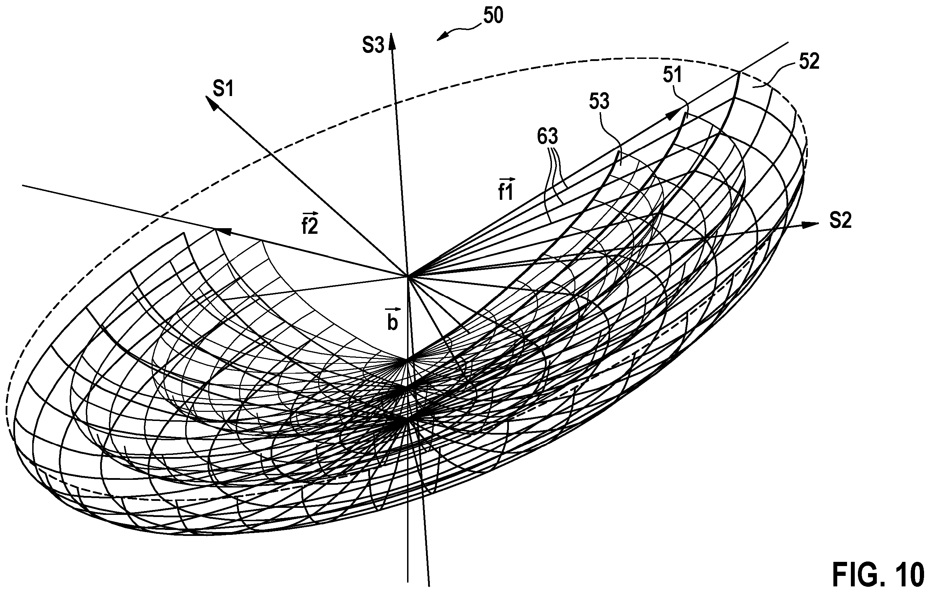

Fig. 10 shows the isoforce area 51 exclusively for pressure forces, corresponding to a half space of the complete isoforce area 51 as shown in Fig. 9. During operation, only pressure forces from the tissue 13 to the functional electrode 2 are to be expected. Furthermore, Fig. 10 shows an isoforce area 52 of a higher force and an isoforce area 53 of a lower force. The isoforce areas 51, 52, 53 are shown cut in the gap of the three vectors $\overrightarrow{f1}$, $\overrightarrow{f2}$ and $\vec{b}$.

In the areas covered by $\overrightarrow{f1}$ and $\vec{b}$ and also $\overrightarrow{f2}$ and $\vec{b}$, if the isoforce areas 51, 52, 53 are visible when cut. The rays 63 emanating from the zero point correspond to the edges of cut cones. All signal vectors $\vec{S}$ are generally located on a specific cone, since they always start from zero. Signal vectors $\vec{S}$ which lie on the same cone represent forces that act at the same angle $\alpha$ from the tissue 13 on the functional electrode 2, but may have different amounts and $\varphi$ directions.

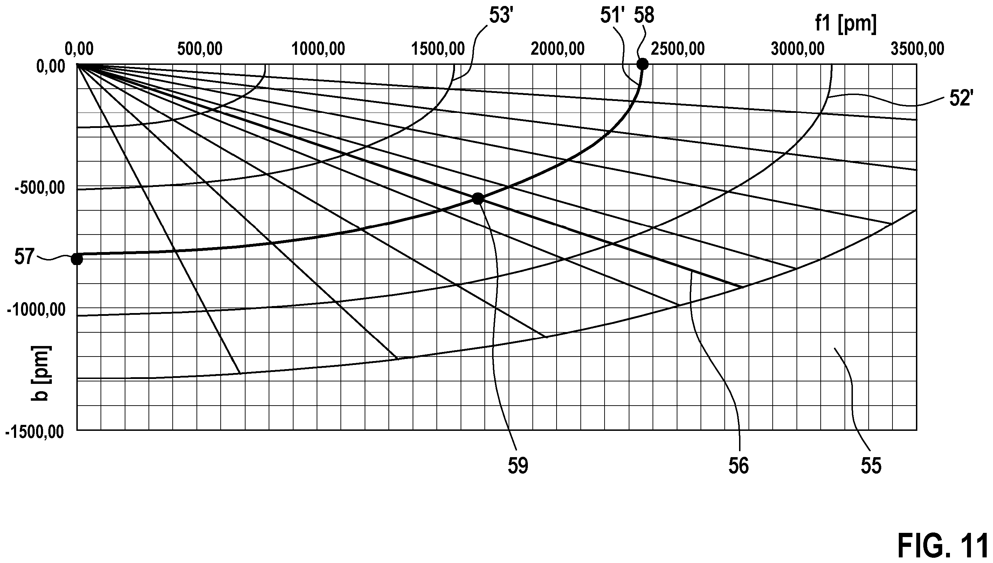

Fig. 11 shows a two-dimensional characteristic diagram 55 of the measuring device. The characteristic diagram 55 is obtained by orthogonalising the vector plane $\vec{f1}$ and $\vec{b}$ as shown in Fig. 10. With $\vec{f2}$ and $\vec{b}$ it is possible to generate an equivalent characteristic diagram, because $|\vec{f1}| = c \cdot |\vec{f2}|$, therefore, a fixed relationship exists. The curves 51', 52', 53' correspond to the lines of intersection of the cut isoforce areas 51, 52, 53. The line 56, for example, corresponds to the conical cut, which is the same for all lines of action 42 of the tissue force $\vec{F_G}$ (Fig. 8), which act on the functional electrode 2 at an angle $\alpha = 45°$. The point of intersection 59 of the curves 51', 56 represents a force $F_G = 30$ g, which acts on the functional electrode 2 at an angle $\alpha = 45°$. The point 57 marks the magnitude of the vector b with $F_G = 30$ g and $\alpha = 0°$, that is to say $b_{30g,0°}$. Point 58 identifies the magnitude of the vector $\vec{f1}$ with $F_G = 30$ g and $\alpha = 90°$, that is to say $f1_{30g,90°}$.

To determine $F_G$ and $\alpha$, the coordinates f1 and b in the characteristic diagram 55 are first calculated from the underlying signal vector $\vec{S}$ via the signal-force determination algorithm. With these values the result is:

$$F_G = F_c \cdot \sqrt{\left(\frac{f1}{f1_{Fc,90°}}\right)^2 + \left(\frac{b}{b_{Fc,0°}}\right)^2}$$

and

$$for\ b \neq 0, \qquad \alpha = tan^{-1}\left(\frac{f1}{b} \cdot \frac{b_{Fc,0°}}{f1_{Fc,90°}}\right)$$

*für b = 0 ist α = 90°*

If $\alpha$ assumes a negative value, the force determined is a tensile force.

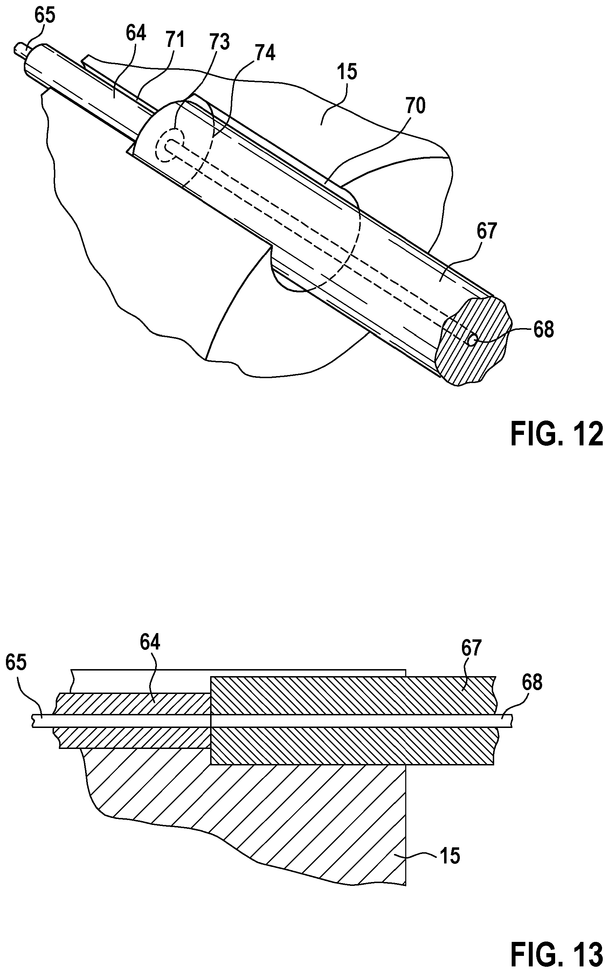

Fig. 12 and 13 show the transducer body 15 in the region of a connection point between a portion 64 of the glass fibre 16 and a pigtail 67 connected to it in the form of a separate glass fibre in addition to the glass fibre 16.

Fig. 12 shows a detail of a transducer body 15 in a perspective view. A groove 70 is used to align and fix the pigtail 67, while a structure 71 aligns the portion 64 of the glass fibre 16 relative thereto. The glass fibre 16 and the pigtail 67, which serves as the feed line for the glass fibre 16, may have the same diameter or different diameters.

A core 65 of the glass fibre 16 and a core 68 of the pigtail 67 are brought into alignment with the geometries of the groove 70 and the structure 71 by aligning the outer radii 73, 74 of the glass fibre 16 on the one hand and the pigtail 67 on the other hand, so that light may be coupled with low loss from the pigtail 67 into the glass fibre 16 used for signal detection and vice versa.

Fig. 13 shows a longitudinal sectional view of the glass fibre 16 and the transducer body 15 in the region of the connection point. The alignment of the core 65 of the glass fibre 16 and the core 68 of the pigtail 67 relative to each other is easily achieved by positioning on the transducer body 15.

List of reference signs

| | |
|---|---|
| 1 | catheter device |
| 2, 2' | functional electrode |
| 3, 3' | sealing element |
| 4 | rigid segment |
| 5 | ring electrode |
| 6 | controllable segment |
| 7 | catheter shaft |
| 8 | control button |
| 9 | handle |
| 10 | optical connection |
| 11 | electrical connection |
| 12 | flush connection |
| 13 | tissue |
| 14 | insulation layer |
| 15 | transducer body |
| 150-153 | transducer portions |
| 154-156 | connecting portions |

| 16 | optical fibre (glass fibre) |
|---|---|
| 17 | optical grating |
| 18 | optical grating |
| 19 | optical grating |
| 20 | protective sleeve |
| 21 | flushing tube |
| 22 | cooling channel |
| 23 | electrical cable to the functional electrode |
| 24 | cable channel |
| 25 | connection transducer body-protective cover |
| 26 | soldering point |
| 27 | recess (in the transducer body) |
| 28 | flushing channels |
| 29 | flushing openings |
| 30 | connection transducer body-functional electrode |
| 31 | connection transducer body-sealing element |
| 32 | connection protective sleeve-sealing element |
| 33 | recess |
| 34 | channel |
| 35 | recess |
| 36 | recess |
| 38 | channel |
| 39 | connection transducer body-flushing tube |
| 40 | stop |
| 41 | functional electrode axis |
| 42 | line of action |
| 43 | centre |
| 44-48 | calibration point |
| 50 | signal diagram |
| 51 | isoforce area |
| 51' | isoforce curve |
| 52 | isoforce area of a larger force |
| 52' | isoforce curve of a larger force |
| 53 | isoforce area of a lower force |
| 53' | isoforce curve of a lower force |
| 55 | characteristic diagram (2D and orthogonally directed) |
| 56 | iso-angle straight line |
| 57 | magnitude of the signal vector at calibration point 1 |
| 58 | magnitude of the signal vector at calibration point 5 |
| 59 | reference coordinate of the calibration range |
| 60 | thermocouple |
| 61 | channel |
| 62 | air gap |
| 63 | conic sections |
| 64 | portion |
| 65 | core |
| 67 | pigtail |
| 68 | core |
| 70 | groove |
| 71 | structure |
| 73 | outer diameter of the sensory glass fibre |
| 74 | outer diameter of the supply fibre |
| $\alpha$ | elevation angle (angle line of action-functional electrode axis) |
| $\phi$ | azimuth angle (angle of rotation around functional electrode axis) |
| L | longitudinal axis |

**Claims**

1. A catheter device (1), comprising a measuring device for measuring a force effect on the catheter device (1), the measuring device comprising an elastically deformable transducer body (15) having a plurality of transducer portions (150-153) and a sensor device arranged on the transducer body (15), the transducer portions (150-153) forming at least one pair of adjacent transducer portions (150-153), which are connected to each other via a connecting portion (154-156) and are movable relative to each other with deformation of the connecting portion (154-156), and the sensor device being designed to measure a force effect between the transducer portions (150-153) of the at least one pair of adjacent transducer portions (154-156), **characterised in that** the transducer body (15) is made of a glass material, whereby the glass material is a metastable basically amorphous structure with low crystallinity comprising silicon dioxide .

2. The catheter device (1) according to claim 1, **characterised in that** the sensor device comprises an optical fibre (16) arranged on the transducer body (15), said optical fibre (16) comprising at least one optical grating (17, 18, 19) for measuring a force effect between the transducer portions (150-153) of the at least one pair of adjacent transducer portions (154-156).

3. The catheter device (1) according to claim 2, **characterised in that** the optical fibre (16) is formed by a glass fibre.

4. The catheter device (1) according to claim 2 or 3, **characterised in that** the optical fibre (16) is connected to the transducer portions (150-153) of the transducer body (15) by bonding, glass soldering, eutectic soldering or welding.

5. The catheter device (1) according to one of claims 2 to 4, **characterised by** a pigtail (67), which is connected to the transducer body (15), is separate from the optical fibre (16), and is optically coupled to the optical fibre (16) at a connection point on the transducer body (15).

6. The catheter device (1) according to one of the preceding claims, **characterised in that** a plurality of transducer portions (150-153) are arranged in a line along a longitudinal axis (L).

7. The catheter device (1) according to claim 6, **characterised in that** the connecting portion (154-156) of at least one pair of adjacent transducer portions (150-153) is bendable about a defined spatial direction extending transversely to the longitudinal axis (L) in such a way that the transducer portions (150-153) of the at least one pair of adjacent transducer portions (150-153) are movable relative to each other about the defined spatial direction via the connecting portion (154-156).

8. The catheter device (1) according to claim 6 or 7, **characterised in that** the transducer body (15) forms at least two pairs of adjacent transducer portions (150-153), which are each connected to each other via a connecting portion (154-156) and are movable relative to each other about different spatial directions extending transversely to the longitudinal axis (L).

9. The catheter device (1) according to one of the preceding claims, **characterised in that** the transducer body (15) has a cooling channel (22) for conducting a coolant.

10. The catheter device (1) according to one of the preceding claims, **characterised by** a protective sleeve (20) in which the transducer body (15) is arranged and to which the transducer body (15) is firmly connected.

11. The catheter device (1) according to claim 10, **characterised by** a gap arranged radially between the transducer body (15) and the protective sleeve (20).

12. The catheter device (1) according to claim 10 or 11, **characterised in that** the transducer body (15) is firmly connected at a first end to the protective sleeve (20) and is movable with a second end, which is remote from the first end, relative to the protective sleeve (20).

13. The catheter device (1) according to claim 12, **characterised by** a functional electrode (2) for introducing an electrical signal into tissue (13), which functional electrode is arranged at the second end of the transducer body (15).

14. The catheter device (1) according to claims 12 or 13, **characterised by** an elastically deformable sealing element (3) for sealing a transition between the protective sleeve (20) and the second end of the transducer body (15).

**15.** The catheter device (1) according to one of claims 10 to 14, **characterised in that** the protective sleeve (20) forms a stop for limiting elastic deformation of the transducer body (15).

**Patentansprüche**

**1.** Kathetervorrichtung (1), umfassend eine Messvorrichtung zum Messen einer auf die Kathetervorrichtung (1) einwirkenden Kraft, wobei die Messvorrichtung einen elastisch verformbaren Wandlerkörper (15) mit mehreren Wandlerabschnitten (150-153) und eine auf dem Wandlerkörper (15) angeordnete Sensorvorrichtung umfasst, wobei die Wandlerabschnitte (150-153) wenigstens ein Paar benachbarter Wandlerabschnitte (150-153) bilden, die über einen Verbindungsabschnitt (154-156) miteinander verbunden sind und bei Verformung des Verbindungsabschnitts (154-156) relativ zueinander beweglich sind, und wobei die Sensorvorrichtung dazu ausgelegt ist, eine Krafteinwirkung zwischen den Wandlerabschnitten (150-153) des wenigstens einen Paares benachbarter Wandlerabschnitte (154-156) zu messen, **dadurch gekennzeichnet, dass** der Wandlerkörper (15) aus einem Glasmaterial besteht, wobei es sich bei dem Glasmaterial um eine metastabile, im Wesentlichen amorphe Struktur mit geringer Kristallinität handelt, die Siliziumdioxid umfasst.

**2.** Kathetervorrichtung (1) nach Anspruch 1, **dadurch gekennzeichnet, dass** die Sensorvorrichtung eine auf dem Wandlerkörper (15) angeordnete optische Faser (16) umfasst, wobei die optische Faser (16) wenigstens ein optisches Gitter (17, 18, 19) zum Messen einer Krafteinwirkung zwischen den Wandlerabschnitten (150-153) des wenigstens einen Paares benachbarter Wandlerabschnitte (154-156) umfasst.

**3.** Kathetervorrichtung (1) nach Anspruch 2, **dadurch gekennzeichnet, dass** die optische Faser (16) aus einer Glasfaser gebildet ist.

**4.** Kathetervorrichtung (1) nach Anspruch 2 oder 3, **dadurch gekennzeichnet, dass** die optische Faser (16) durch Kleben, Glaslöten, Eutektiklöten oder Schweißen mit den Wandlerabschnitten (150-153) des Wandlerkörpers (15) verbunden ist.

**5.** Kathetervorrichtung (1) nach einem der Ansprüche 2 bis 4, **gekennzeichnet durch** eine Anschlusslitze (67), die mit dem Wandlerkörper (15) verbunden ist, von der optischen Faser (16) getrennt ist und an einem Verbindungspunkt am Wandlerkörper (15) optisch mit der optischen Faser (16) gekoppelt ist.

**6.** Kathetervorrichtung (1) nach einem der vorangehenden Ansprüche, **dadurch gekennzeichnet, dass** mehrere Wandlerabschnitte (150-153) in einer Linie entlang einer longitudinalen Achse (L) angeordnet sind.

**7.** Kathetervorrichtung (1) nach Anspruch 6, **dadurch gekennzeichnet, dass** der Verbindungsabschnitt (154-156) wenigstens eines Paares benachbarter Wandlerabschnitte (150-153) um eine definierte Raumrichtung, die sich quer zur longitudinalen Achse (L) erstreckt, derart biegbar ist, dass die Wandlerabschnitte (150-153) des wenigstens einen Paares benachbarter Wandlerabschnitte (150-153) über den Verbindungsabschnitt (154-156) relativ zueinander um die definierte Raumrichtung beweglich sind.

**8.** Kathetervorrichtung (1) nach Anspruch 6 oder 7, **dadurch gekennzeichnet, dass** der Wandlerkörper (15) wenigstens zwei Paare benachbarter Wandlerabschnitte (150-153) bildet, die jeweils über einen Verbindungsabschnitt (154-156) miteinander verbunden sind und relativ zueinander um verschiedene Raumrichtungen beweglich sind, die sich quer zur longitudinalen Achse (L) erstrecken.

**9.** Kathetervorrichtung (1) nach einem der vorangehenden Ansprüche, **dadurch gekennzeichnet, dass** der Wandlerkörper (15) einen Kühlkanal (22) zum Leiten eines Kühlmittels aufweist.

**10.** Kathetervorrichtung (1) nach einem der vorangehenden Ansprüche, **gekennzeichnet durch** eine Schutzhülse (20), in der der Wandlerkörper (15) angeordnet ist und mit der der Wandlerkörper (15) fest verbunden ist.

**11.** Kathetervorrichtung (1) nach Anspruch 10, **gekennzeichnet durch** einen radial zwischen dem Wandlerkörper (15) und der Schutzhülse (20) angeordneten Spalt.

**12.** Kathetervorrichtung (1) nach Anspruch 10 oder 11, **dadurch gekennzeichnet, dass** der Wandlerkörper (15) an einem ersten Ende fest mit der Schutzhülse (20) verbunden ist und mit einem zweiten Ende, das vom ersten Ende entfernt ist, relativ zur Schutzhülse (20) beweglich ist.

13. Kathetervorrichtung (1) nach Anspruch 12, **gekennzeichnet durch** eine Funktionselektrode (2) zum Einleiten eines elektrischen Signals in Gewebe (13), wobei die Funktionselektrode am zweiten Ende des Wandlerkörpers (15) angeordnet ist.

14. Kathetervorrichtung (1) nach Anspruch 12 oder 13, **gekennzeichnet durch** ein elastisch verformbares Dichtungselement (3) zum Abdichten eines Übergangs zwischen der Schutzhülse (20) und dem zweiten Ende des Wandlerkörpers (15).

15. Kathetervorrichtung (1) nach einem der Ansprüche 10 bis 14, **dadurch gekennzeichnet, dass** die Schutzhülse (20) einen Anschlag zum Begrenzen der elastischen Verformung des Wandlerkörpers (15) bildet.

**Revendications**

1. Dispositif de cathéter (1), comprenant un dispositif de mesure destiné à mesurer un effet de force sur le dispositif de cathéter (1), le dispositif de mesure comprenant un corps de transducteur élastiquement déformable (15) ayant une pluralité de parties de transducteur (150 à 153) et un dispositif de capteur agencé sur le corps de transducteur (15), les parties de transducteur (150 à 153) formant au moins une paire de parties de transducteur (150 à 153) adjacentes, qui sont reliées l'une à l'autre par l'intermédiaire d'une partie de liaison (154 à 156) et sont mobiles les unes par rapport aux autres avec une déformation de la partie de liaison (154 à 156), et le dispositif de capteur étant conçu pour mesurer un effet de force entre les parties de transducteur (150 à 153) de l'au moins une paire de parties de transducteur (154 à 156) adjacentes, **caractérisé en ce que** le corps de transducteur (15) est fabriqué en un matériau de verre, moyennant quoi le matériau de verre est une structure essentiellement amorphe métastable de faible cristallinité comprenant du dioxyde de silicium.

2. Dispositif de cathéter (1) selon la revendication 1, **caractérisé en ce que** le dispositif de capteur comprend une fibre optique (16) agencé sur le corps de transducteur (15), ladite fibre optique (16) comprenant au moins un réseau optique (17, 18, 19) destiné à mesurer un effet de force entre les parties de transducteur (150 à 153) de l'au moins une paire de parties de transducteur (154 à 156) adjacentes.

3. Dispositif de cathéter (1) selon la revendication 2, **caractérisé en ce que** la fibre optique (16) est formée par une fibre de verre.

4. Dispositif de cathéter (1) selon la revendication 2 ou 3, **caractérisé en ce que** la fibre optique (16) est reliée aux parties de transducteur (150 à 153) du corps de transducteur (15) par adhésion, soudage de verre, soudage eutectique ou brasage.

5. Dispositif de cathéter (1) selon l'une des revendications 2 à 4, **caractérisé par** une fibre amorce (67), qui est reliée au corps de transducteur (15), est séparée de la fibre optique (16), et est optiquement couplée à la fibre optique (16) au niveau d'un point de liaison sur le corps de transducteur (15).

6. Dispositif de cathéter (1) selon l'une des revendications précédentes, **caractérisé en ce qu'**une pluralité de parties de transducteur (150 à 153) sont agencées en une ligne le long d'un axe longitudinal (L).

7. Dispositif de cathéter (1) selon la revendication 6, **caractérisé en ce que** la partie de liaison (154-156) d'au moins une paire de parties de transducteur (150 à 153) adjacentes peut être pliée autour d'une direction spatiale définie s'étendant de manière transversale par rapport à l'axe longitudinal (L) d'une façon telle que les parties de transducteur (150 à 153) de l'au moins une paire de parties de transducteur (150 à 153) adjacentes sont mobiles les unes par rapport aux autres autour de la direction spatiale définie par l'intermédiaire de la partie de liaison (154 à 156).

8. Dispositif de cathéter (1) selon la revendication 6 ou 7, **caractérisé en ce que** le corps de transducteur (15) forme au moins deux paires de parties de transducteur adjacentes (150 à 153), qui sont chacune reliées les unes aux autres par l'intermédiaire d'une partie de liaison (154 à 156) et sont mobiles les unes par rapport aux autres autour de directions spatiales différentes s'étendant transversalement par rapport à l'axe longitudinal (L).

9. Dispositif de cathéter (1) selon l'une des revendications précédentes, **caractérisé en ce que** le corps de transducteur (15) a un canal de refroidissement (22) pour faire passer un liquide de refroidissement.

**10.** Dispositif de cathéter (1) selon l'une des revendications précédentes, **caractérisé par** un manchon protecteur (20) dans lequel le corps de transducteur (15) est agencé et auquel le corps de transducteur (15) est fermement relié.

**11.** Dispositif de cathéter (1) selon la revendication 10, **caractérisé par** un intervalle agencé de manière radiale entre le corps de transducteur (15) et le manchon protecteur (20).

**12.** Dispositif de cathéter (1) selon la revendication 10 ou 11, **caractérisé en ce que** le corps de transducteur (15) est fermement relié au niveau d'une première extrémité au manchon protecteur (20) et est mobile avec une seconde extrémité, qui est distante de la première extrémité, par rapport au manchon protecteur (20).

**13.** Dispositif de cathéter (1) selon la revendication 12, **caractérisé par** une électrode fonctionnelle (2) destinée à introduire un signal électrique dans un tissu (13), laquelle électrode fonctionnelle est agencée au niveau de la seconde extrémité du corps de transducteur (15).

**14.** Dispositif de cathéter (1) selon les revendications 12 ou 13, **caractérisé par** un élément d'étanchéité élastiquement déformable (3) pour étanchéifier une transition entre le manchon protecteur (20) et la seconde extrémité du corps de transducteur (15).

**15.** Dispositif de cathéter (1) selon l'une des revendications 10 à 14, **caractérisé en ce que** le manchon protecteur (20) forme une butée pour limiter la déformation élastique du corps de transducteur (15).

EP 4 216 855 B1

FIG. 1

FIG. 2

FIG. 3A

FIG. 3B

FIG. 4A

FIG. 4B

FIG. 4C

**FIG. 5**

**FIG. 6**

**FIG. 7**

**FIG. 8**

FIG. 9

EP 4 216 855 B1

S3

S1

50

52

51

53

63

f⃗2

f⃗1

S2

b⃗

FIG. 10

EP 4 216 855 B1

FIG. 11

EP 4 216 855 B1

FIG. 12

FIG. 13

**REFERENCES CITED IN THE DESCRIPTION**

**Patent documents cited in the description**

- US 20140081264 A1
- US 20060200049 A1
- WO 2013177577 A2
- US 2015359484 A1